(19) **Europäisches Patentamt**
**European Patent Office**
**Office européen des brevets**

(11) **EP 2 876 099 B1**

(12) **EUROPEAN PATENT SPECIFICATION**

(45) Date of publication and mention
of the grant of the patent:
**15.11.2017 Bulletin 2017/46**

(51) Int Cl.:
*C07C 7/12* *(2006.01)*  *C07C 7/20* *(2006.01)*
*C07C 13/39* *(2006.01)*  *C23C 16/40* *(2006.01)*

(21) Application number: **13306591.2**

(22) Date of filing: **21.11.2013**

(54) **Norbornadiene purification method**

Norbornadienreinigungsverfahren

Procédé de purification de norbornadiène

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.05.2015 Bulletin 2015/22**

(73) Proprietor: **L'AIR LIQUIDE, SOCIETE ANONYME
POUR
L'ETUDE ET L'EXPLOITATION DES PROCEDES
GEORGES CLAUDE
75007 Paris (FR)**

(72) Inventors:
• **Danvel, Laurent
21630 POMMARD (FR)**
• **Plaza, Sonia
71100 CHALON SUR SAONE (FR)**

(74) Representative: **Grout de Beaufort,
François-Xavier
L'Air Liquide S.A.
Intellectual Property Department
75, Quai d'Orsay
75321 Paris Cedex 07 (FR)**

(56) References cited:
**US-A1- 2009 159 843    US-B2- 6 846 515**

**Description**

The present invention concerns new porogen compositions and a new method for improving BCHD (Norbornadiene, BisCycloHeptaDiene) purity by removing the benzene usually present. Benzene being a carcinogenic material, the purified compounds has a reduced toxicity.

Purified norbornadiene can then be used to in vapor deposition methods such as (plasma-enhanced) chemical vapor deposition for semiconductor manufacturing.

In semiconductor and electronics industries, device shrinking allows higher performances and processing speed.

However reducing the device dimensions led to an increase of defects like leakage current through the insulators or increase capacitance in BEOL leading to slower chips speed.

As components have shrinked and transistors are closer, the insulating dielectrics (low-k) have thinned to the point where charge build up and crosstalk negatively affect the device performances. Replacing the $SiO_2$ or $SiO_2$:F with a low dielectric constant (k) that is know a porous SiOC film of the same thickness reduces parasitic capacitance, enabling faster switching speeds and lower heat dissipation.

Those porous low-k films are traditionally deposited using $SiC_xO_yH_z$ containing precursors such as for instance, DEOMS, DEMS, 4MS, 3MS, OMCTS, TMCTS in combination with a porogen such as ATPR, BCHD or $C_xH_z$ where x, y and z are integers.

Additional gases such as $O_2$, $N_2$, $N_2O$, $NO_2$, NO, Ar or other inert gases can be introduced to tune the process. The porogen will be incorporated into the film and after deposition the film may be cured (exposed to UV or a remote plasma) for less than 10 minutes.

During the curing time, the porogen is removed from the films leaving voids called pores that allow a decrease of the dielectric constant of the film (k or air = 1). Examples of UV curing processes can be found in US6756085. US20090159843 describes compositions comprising Norbornadiene and a stabilizer. BCHD and benzene have very close boiling point and can not be removed by standard distillation (Benzene BP=80,1°C - BCHD BP=89°C).

Benzene presents however serious safety risks causing cancer or other illness. It is also an impurity in the BCHD that is preferred to be used in ultrahigh purity in microelectronics to enhance yield of processes and limit process variations.

By the term benzene-free BCHD the applicant means BCHD that containing residual amount of benzene, possibly undetected. It means benzene content below 10ppm, and more preferably below 1 ppm.

The present invention provides a method of removing benzene from raw BCHD. This allows a production of a benzene free BCHD that is safer to handle and could enhance yield control.

Activated carbon is known to adsorb benzene in water and in air:

- ESPR - Environ Sci & Pollut Res 10(1) 6-8 (2003) - Kinetics of Benzene adsorption onto Activated carbon.
- GCMC Simulations in Zeolite MFI and Activated Carbon for Benzene Removal from Exhaust Gaseous Streams - P.Cosoli et al.

For liquid-phase adsorption on activated carbon of a solute in water, Dubinin-Radushkevich equation can be expressed as followed:

$$q = q_{max} \exp\left[-\left(\frac{RT \ln \frac{C_s}{C_e}}{\beta E_0}\right)^2\right]$$

Where :

q is the amount adsorbed
$q_{max}$ is the maximum capacity in the pore
$\beta$ is called the affinity coefficient and expresses the ratio of the characteristic free energies of adsorption for the test and for a reference. $\beta$ is affected by the adsorbate properties such as size and shape, boiling point and chemistry.
$E_0$ is the characteristic adsorption energy for a reference on a specific adsorbent.
$C_s$ is the solute solubility
$C_e$ is the equilibrium concentration
R is the universal gas constant

T is the temperature.

Supposing that in raw material, only BCHD and benzene can be adsorbed on activated carbon. Adsorption selectivity will depend on the affinity coefficients of BCHD and Benzene.

According to S.Ismadji and S.K.Bhatia, for gas, the affinity coefficient β can be expressed:

$$\beta = 0{,}1303 + 0{,}0353 P_e$$

With $P_e$ molar polarization

$$P_e = \left[ \frac{n_D^2 - 1}{n_D^2 + 2} \right] \frac{M}{\rho_L}$$

Where :

$n_D$ is refracting index
M : Molecular weight
ρ : liquid density.

For liquid-phase adsorption, β are about half of those for the gas-phase.

Thus, in liquid-phase adsorption: $\beta_{BCHD} = 0{,}57$ and $\beta_{Benzene} = 0{,}53$.

Affinity coefficient of benzene and BCHD are very close. BCHD appears more polarizable than benzene. Indeed, both molecules are cyclic, but, BCHD possesses one carbon more than benzene.

Besides, the molecule has four carbons sp2 and three carbons sp3 whereas benzene contains six carbons sp2 which are more electronegative than sp3 carbons.

Therefore, according to affinity coefficient, it seems that there should be a selective adsorption of BCHD on activated carbon. In this case, benzene removal in raw material would not be possible.

Nevertheless, there is a need for providing benzene free BCHD as it is mentioned above.

The inventors of the present invention show that, surprisingly, activated carbon can be used to remove benzene from BCHD.

Activated carbon can be used on BCHD not causing decomposition nor contamination of the raw material while ensuring a complete adsorption of the present benzene.

Moreover, it can greatly increase the final purity of the product, a critical parameter for semiconductor manufacturing.

The object of the present invention is a porogen composition comprising for 100% by weight:

- at least 99,5% by weight of norbornadiene ;
- from 10 ppm to 500 ppm by weight of a nitroxyl radical based stabilizer;
- from 250 ppb to 10 ppm by weight of benzene.

According to other embodiments, the present invention concerns also:

A composition as defined above comprising less than 1 ppm of benzene.

A composition as defined above comprising at least 99,95% of norbornadiene.

A composition as defined above wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO), di-tert-butyl nitroxyl, 1-oxyl-2,2,6,6-tetramethylpiperidin-4-one, 1-oxyl-2,2,6,6-tetramethylpiperidin-4-yl acetate, 1-oxyl-2,2,6,6-tetramethylpiperidin-4-yl 2-ethylhexanoate, 1-oxyl-2,2,6,6-tetramethylpiperidin-4-yl stearate, 1-oxyl-2,2,6,6-tetramethylpiperidin-4-yl benzoate, 1-oxyl-2,2,6,6-tetramethylpiperidin-4-yl 4-tert-butylbenzoate, bis(1-oxyl-2,2,6,6-tetramethylpiperidin-4-yl)succinate, bis(1-oxyl-2,2,6,6-tetramethylpiperidin-4-yl)adipate, bis(1-oxyl-2,2,6,6-tetramethylpiperidin-4-yl)sebacate, bis(1-oxyl-2,2,6,6-tetramethylpiperidin-4-yl)n-butylmalonate, bis(1-oxyl-2,2,6,6-tetramethylpiperidin-4-yl)phthalate, bis(1-oxyl-2,2,6,6-tetramethylpiperidin-4-yl)isophthalate, bis(1-oxyl-2,2,6,6-tetramethylpiperidin-4-yl)terephthalate, bis(1-oxyl-2,2,6,6-tetramethylpiperidin-4-yl)hexahydroter-ephthalate, N,N'-bis(1-oxyl-2,2,6,6-tetrame-

thylpiperidin-4-yl)adipamide, N-(1-oxyl-2,2,6,6-tetramethylpiperidin-4-yl)-caprolactam, N-(1-oxyl-2,2,6,6-tetramethylpiperidin-4-yl)-dodecylsuccinimide, 2,4,6-tris(1-oxyl-2,2,6,6-tetrame-thylpiperidin-4-yl) isocyanurate, 2,4,6-tris-[N-butyl-N-1-oxyl-2,2,6,6-tetramethylpiperidin-4-yl]-s-triazine, 4,4'-ethyl-enebis(1-oxyl-2,2,6,6-tetramethylpiperazin-3-one) and combinations thereof.

A composition as defined above, wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO) and combinations thereof.

Another object of the present invention is a method of preparation of a composition as defined above comprising:

Step a) providing a benzene containing norbornadiene solution comprising more than 100 ppm of benzene,
Step b) passing the benzene containing norbornadiene solution through an adsorbent such as activated carbon bed, zeolite alumina or a combination thereof,
Step c) adding to the norbornadiene solution issued from step b) a nitroxyl radical based stabilizer.

According to other embodiments, the present invention also concerns:

A method as defined above, wherein the adsorbent is an activated carbon bed.

A method as defined above, wherein the activated carbon bed contains pores and wherein the pores have an equivalent spherical diameter comprised between 0,4 nm and 50 nm.

A method as defined above, wherein the activated carbon is used at a temperature ranging from 0°C to 100°C, preferably 20°C to 50°C, and a pressure ranging from 80 kPa to 665 kPa, preferably between 133 kPa to 200 kPa.

A method as defined above, wherein the activated carbon is initially baked at temperatures ranging from 30°C up to 300°C, preferably below 250°C.

A method as defined above, wherein the activated carbon used is regenerated by baking at a temperature comprised between 50°C and 400°C before reutilization.

The method as defined above, wherein the treated norbornadiene issued from step b) is then purified by standard distillation to obtain a final product with a purity greater than 99,9%, preferably greater than 99,95%.

According to another embodiment, the baking and the filtration process of the method of the invention are done under inert conditions, using inert gases such as He, Ar or $N_2$, and possibly at non-atmospheric pressure.

According to another embodiment, the separation of benzene from BCHD is done by the mean of extractive distillation.

According to another embodiment, the separation of benzene from BCHD is done by combining extractive distillation with an adsorbent.

Another object of the present invention is the use of a composition as defined above in a vapour deposition process for producing a porous organosilica glass film represented by the formula $Si_vO_wC_xH_yF_z$, where v+w+x+y+z = 100%, v is from 10 to 35 atomic%, w is from 10 to 65 atomic%, x is from 5 to 30 atomic%, y is from 10 to 50 atomic% and z is from 0 to 15 atomic%, said method comprising:

- providing a substrate within a vacuum chamber;
- introducing into the vacuum chamber gaseous reagents including at least one precursor selected from the group consisting of an organosilane and an organosiloxane, and a porogen composition, as defined above, distinct from the precursor;
- applying energy to the gaseous reagents in the vacuum chamber to induce reaction of the gaseous reagents to deposit a preliminary film on the substrate, wherein the preliminary film contains the porogen; and
- removing from the preliminary film substantially all of the labile organic material to provide the porous film with pores and a dielectric constant less than 2,6.

Preferably the dielectric constant is less than 2,2.

According to other embodiments, the present invention also concerns:

The use as defined above, wherein the energy is plasma energy and the porogen is removed by exposure to ultraviolet radiation.

The use as defined above, wherein the at least one precursor is a member selected from the group consisting of diethoxymethylsilane, dimethoxymethylsilane, di-isopropoxymethylsilane, di-t-butoxymethylsilane,methyltriethoxysilane, methyltrimethoxysilane, methyltriisopropoxysilane, methyltri-t-butoxysilane, dimethyldimeth-oxysilane, dimethyldiethoxysilane, dimethyldi-isopropoxysilane, dimethyldi-t-butoxysilane, and tetraethoxysilane. Preferably, the precursor is DEMS.

The effectiveness of activated carbon to remove benzene from BCHD raw material could be due to the pore size distribution in activated carbon and to the structural difference between benzene and BCHD. It is supposed that benzene which is a planar molecule can be adsorbed in pores where BCHD uptake is not possible.

Activated carbon that can be used for BCHD purification contains pore size ranging from 0,4nm to 50nm.

In alternative to activated carbon, zeolites or alumina could be used. But advantageously, activated carbon is preferred.

In extractive distillation, an additional solvent, separating agent, with a high boiling point, is used to alter the relative volatility of the components to be separated.

In this way, it is possible to obtain one pure component at the top of the column and the other, together with the solvent at the bottom.

The distillation can be performed on a batch column.

If BCHD is retained in the boiler with the solvent, BCHD can be purified after the extractive distillation on the same column. The separation would be easy due to the high boiling point of the solvent.

If benzene is retained in the boiler with the solvent, according to the cost of the separating agent, one can choose to purify the solvent on the same column after the extractive distillation.

When performing extractive distillation between benzene and BCHD, the following solvents can be used: isoamyl formate, dimethylformamide, dimethylsulfoxide, ethyl acetoacetate, methyl acetoacetate, ethyl acetate, phenol, butyl phenol, 4-nitro phenol, 2-furaldehyde, isobutyl butyrate, cyanimide solvents.

Example of application of extractive distillation can be found in US 5 458 741. This patent studies the rise of relative volatility between benzene and cyclohexene using these extractive agents.

The effectiveness of these chemicals to perform extractive distillation between benzene and BCHD is not obvious.

Indeed:

- BCHD is a diene and cyclohexene contains only one double bond. Thus, between BCHD and cyclohexene, BCHD is functionally closer than benzene.
- US 5 458 741 studies the possibility to increase the relative volatility to obtain a final purity of 99% purifying a mixture containing 80% of benzene and 20% of cyclohexene. However, obtaining ultra-pure chemicals (purity > 99%) requires a number of theoretical plates more important.

Therefore, the extractive agents should increase significantly the relative volatility between BCHD and benzene in order to perform distillation with a number of theoretical plates between 10 and 40.

## Examples

Example 1 - Activated carbon:

184g of benzene-containing BCHD solution (containing more than 400 ppm) were placed into a separating funnel containing 49,7g of activated carbon and sand and Teflon cotton.

Twelve fractions of filtered product were taken and analyzed by GC.

Results obtained are as follow:

| Fraction | Mass (g) of extracted product | Benzene detected |
|----------|-------------------------------|------------------|
| 1 | 11.5 | 0 |
| 2 | 13 | 0 |
| 3 | 11 | 0 |
| 4 | 12.5 | 0 |
| 5 | 12 | 0 |
| 6 | 11.5 | 0 |
| 7 | 14.5 | 0 |
| 8 | 12.5 | 0 |
| 9 | 13.5 | 0 |

(continued)

| Fraction | Mass (g) of extracted product | Benzene detected |
|---|---|---|
| 10 | 14 | 0 |
| 11 | 13 | 0 |
| 12 | 11 | 0 |

Benzene containing BCHD : 184g
Total weight of filtered material : 150g
% of loss : 100*(184-150)/184 = 18 %

Funnel was then refilled with 187g of benzene-containing BCHD to test the total adsorption capacity.

| Fraction | Product Fraction Mass (g) | Benzene detection |
|---|---|---|
| 1 | 10.5 | 0 |
| 2 | 12 | 0 |
| 3 | 12.5 | 0 |
| 4 | 13.0 | 0 |
| 5 | 12.5 | 0 |
| 6 | 12.0 | 0 |
| 7 | 10.5 | 1 |
| 8 | 14.5 | 1 |
| 9 | 15 | 1 |
| 10 | 20.5 | 1 |
| 11 | 25.5 | 1 |
| 12 | 22.0 | 1 |

Benzene containing BCHD : 187g
Total weight of filtered material : 180.5g
% of loss : 100*(187-180.5)/187 = 3,4 %

Benzene is apparent at sample 7, this indicated that 222,5g of benzene-containing BCHD could be filtered before saturation using 49,7g of activated carbon.

Example 2 - Activated carbon :

A second experiment was performed using 50,8g of activated coal. For the first saturation trial, 366g of benzene-containing BCHD were added. No benzene was detected after extraction of 330g of filtered product (10% of loss). 210g of benzene-containing BCHD were reintroduced. The first five extractions (total 89g) showed no benzene. The 115g of product extracted afterwards indicated traces of benzene (loss for this second pass: 3%). Therefore, 419g of product were filtered using 50,8g of activated carbon (with 42g of product loss).

Example 3 - Activated carbon :

A third experiment was performed. The activated carbon was pre-baked at 150°C during approximately 15 hours. 50g of activated carbon were used. 379g of benzene-containing BCHD were filtered. 345g of product were extracted (loss at 6,6% only) and no benzene was observed at all. 191g of benzene-containing BCHD were used for refill, 188g were extracted (loss at 1,6%). No benzene was detected. Another refill was performed using 203g of benzene-containing BCHD. The 61g extracted first did not show BCHD peaks. The other extracted samples showed some traces of BCHD indicating a saturation of the activated carbon.
In summary, with the 50g of baked activated carbon, 594g of product were filtered with a loss <5%.

Example 4 - Activated carbon :

A fourth experiment was performed. The 50g of activated carbon were regenerated by baking during 6h at 100°C and 48h at 200°C.

After this treatment, the activated carbon was found to re-become effective for benzene adsorption.

Example 5 - Benzene-free distillation

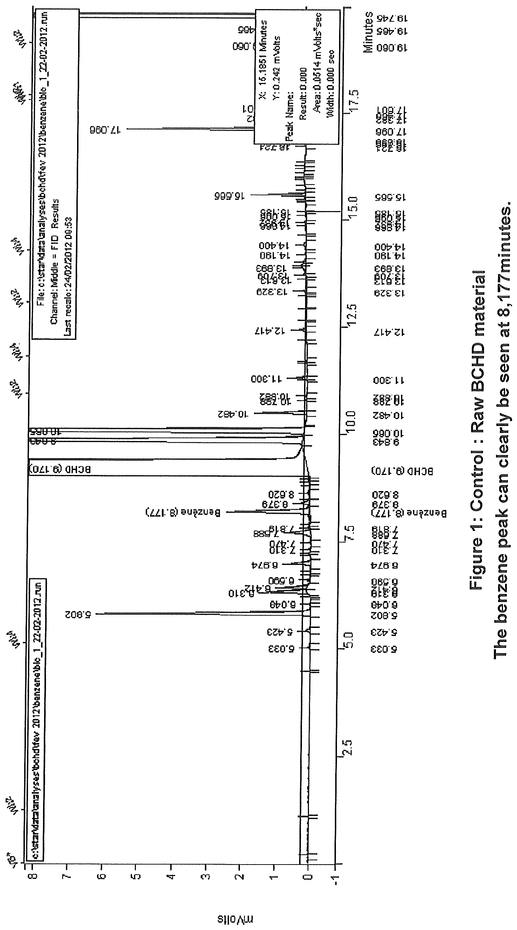

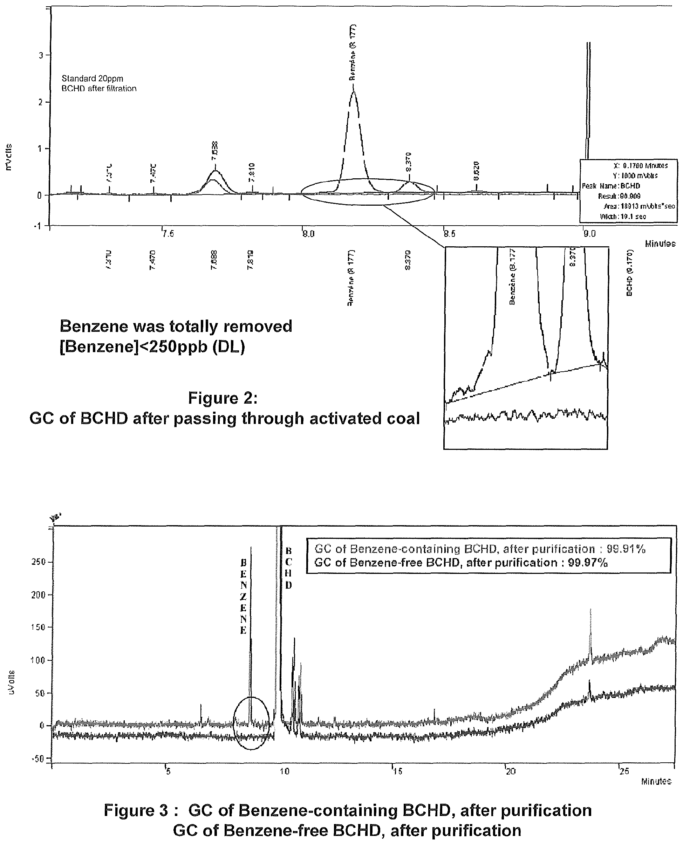

After benzene removal by using activated carbon, a BCHD sample was purified by standard distillation and compared with benzene-containing BCHD (still containing more than 400 ppm) distillation. Benzene-containing BCHD distilled had a final purity of 99,91% (see Figure 3) and in average reachs 99,89% (table 1).

Benzene-free BCHD distillation had a final purity of 99,97% (Figure 3), therefore the activated coal treatment greatly improved the final purity of the product.

Example 6: extractive distillation:

A batch distillation column is used. Thus, the solvent is introduced in the boiler with BCHD raw material. The solvent changes the relative volatility of benzene and BCHD and retains more likely benzene in the boiler.

The result of this distillation is a BCHD without benzene.

Table 1 - Assay result of benzene-containing BCHD in standard distillation.

| distillation # | assay (%) |
|---|---|
| 10 | 99,87 |
| 9 | 99,87 |
| 8 | 99,91 |
| 7 | 99,91 |
| 6 | 99,90 |
| 5 | 99,85 |
| 4 | 99,87 |
| 3 | 99,90 |
| 2 | 99,90 |
| 1 | 99,90 |

Average purity is 99,89% and always below 99,95%.

**Claims**

1. A porogen composition comprising for 100% by weight:

   - at least 99,5% by weight of norbornadiene ;
   - from 10 ppm to 500 ppm by weight of a nitroxyl radical based stabilizer;
   - from 250 ppb to 10 ppm by weight of benzene.

2. Composition according to Claim 1 comprising less than 1 ppm of benzene.

3. Composition of claim 1 or claim 2 comprising at least 99,95% of norbornadiene.

4. Composition according to one of preceding claims wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO), di-tert-butyl nitroxyl, 1-oxyl-2,2,6,6-tetramethylpiperidin-4-one, 1-oxyl-2,2,6,6-tetramethylpiperidin-4-yl acetate, 1-oxyl-2,2,6,6-tetramethylpiperidin-4-yl 2-ethylhexanoate, 1-oxyl-2,2,6,6-tetramethylpiperidin-4-yl stearate, 1-oxyl-2,2,6,6-tetramethylpiperidin-4-yl benzoate, 1-oxyl-2,2,6,6-tetramethylpiperidin-4-yl 4-tert-butylbenzoate, bis(1-oxyl-2,2,6,6-tetramethylpiperidin-4-yl)succinate, bis(1-oxyl-2,2,6,6-tetramethylpiperidin-4-yl)adipate, bis(1-oxyl-2,2,6,6-tetramethylpiperidin-4-yl)sebacate, bis(1-oxyl-2,2,6,6-tetramethylpiperidin-4-yl)n-butylmalonate, bis(1-oxyl-2,2,6,6-tetramethylpiperidin-4-yl)phthalate, bis(1-oxyl-2,2,6,6-tetramethylpiperidin-4-yl)isophthalate, bis(1-oxyl-2,2,6,6-tetramethylpiperidin-4-yl)terephthalate, bis(1-oxyl-2,2,6,6-tetramethylpiperidin-4-yl)hexahydroter-ephthalate, N,N'-bis(1-oxyl-2,2,6,6-tetramethylpiperidin-4-yl)adipamide, N-(1-oxyl-2,2,6,6-tetramethylpiperidin-4-yl)-caprolactam, N-(1-oxyl-2,2,6,6-tetramethylpiperidin-4-yl)-dodecylsuccinimide, 2,4,6-tris(1-oxyl-2,2,6,6-tetramethylpiperidin-4-yl) isocyanurate, 2,4,6-tris-[N-butyl-N-1-oxyl-2,2,6,6-tetramethylpiperidin-4-yl]-s-triazine, 4,4'-ethylenebis(1-oxyl-2,2,6,6-tetramethylpiperazin-3-one) and combinations thereof.

5. Composition of Claim 4, wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO) and combinations thereof.

6. Method of preparation of a composition defined in anyone of claim 1 to 5 comprising:

Step a) providing a benzene containing norbornadiene solution comprising more than 100 ppm of benzene,
Step b) passing the benzene containing norbornadiene solution through an adsorbent such as activated carbon bed, zeolite alumina or a combination thereof,
Step c) adding to the norbornadiene solution issued from step b) a nitroxyl radical based stabilizer.

7. Method according to claim 6, wherein the adsorbent is an activated carbon bed.

8. Method according to claim 7, wherein the activated carbon bed contains pores and wherein the pores have an equivalent spherical diameter comprised between 0,4 nm and 50 nm.

9. Method of claim 7 or 8, wherein the activated carbon is used at a temperature ranging from 0°C to 100°C, preferably 20°C to 50°C, and a pressure ranging from 80 kPa to 665 kPa but preferably between 133 kPa to 200 kPa.

10. Method of one of claims 7 to 9, wherein the activated carbon is initially baked at temperatures ranging from 30°C up to 300°C, preferably below 250°C.

11. Method of one of claims 7 to 10, wherein the activated carbon used is regenerated by baking at a temperature comprised between 50°C and 400°C before reutilization.

12. The method of one of claims 6 to 11, wherein the treated norbornadiene issued from step b) is then purified by standard distillation to obtain a final product with a purity greater than 99,9%, preferably greater than 99,95%.

13. Use of a composition as defined in one of claims 1 to 5 in a vapour deposition process for producing a porous organosilica glass film represented by the formula $Si_vO_wC_xH_yF_z$, where v+w+x+y+z = 100%, v is from 10 to 35 atomic%, w is from 10 to 65 atomic%, x is from 5 to 30 atomic%, y is from 10 to 50 atomic% and z is from 0 to 15 atomic%, said method comprising:

- providing a substrate within a vacuum chamber;
- introducing into the vacuum chamber gaseous reagents including at least one precursor selected from the group consisting of an organosilane and an organosiloxane, and a porogen composition as defined in one of claims 1 to 5 that is distinct from the precursor;
- applying energy to the gaseous reagents in the vacuum chamber to induce reaction of the gaseous reagents to deposit a preliminary film on the substrate, wherein the preliminary film contains the porogen; and
- removing from the preliminary film substantially all of the labile organic material to provide the porous film with pores and a dielectric constant less than 2,6.

14. The use of Claim 13, wherein the energy is plasma energy and the porogen is removed by exposure to ultraviolet radiation.

15. The use of Claim 14, wherein the at least one precursor is a member selected from the group consisting of diethoxymethylsilane, dimethoxymethylsilane, di-isopropoxymethylsilane, di-t-butoxymethylsilane,methyltriethoxysilane, methyltrimethoxysilane, methyltriisopropoxysilane, methyltri-t-butoxysilane, dimethyldimeth-oxysilane, dimethyldiethoxysilane, dimethyldi-isopropoxysilane, dimethyldi-t-butoxysilane, and tetraethoxysilane.

**Patentansprüche**

1. Porogenzusammensetzung, umfassend für 100 Gew%:

   - mindestens 99,5 Gew% Norbornadien;
   - von 10 Gew.ppm bis 500 Gew.ppm eines Nitroxylradikal-basierten Stabilisierungsmittels;
   - von 250 Gew.ppb bis 10 Gew.ppm Benzen.

2. Zusammensetzung nach Anspruch 1, umfassend weniger als 1 ppm Benzen.

3. Zusammensetzung nach Anspruch 1 oder Anspruch 2, umfassend mindestens 99,95 % Norbornadien.

4. Zusammensetzung nach einem der vorhergehenden Ansprüche, wobei das Nitroxylradikal-basierte Stabilisierungsmittel ausgewählt ist aus der Gruppe, bestehend aus 2,2,6,6-Tetramethyl-1-piperidinyloxy (TEMPO), 4-Hydroxy-2,2,6,6-tetramethyl-1-piperidinyloxy (4H-TEMPO), Di-tert-Butylnitroxyl, 1-Oxyl-2,2,6,6-tetramethylpiperidin-4-on, 1-Oxyl-2,2,6,6-tetramethylpiperidin-4-yl-acetat, 1-Oxyl-2,2,6,6-tetramethylpiperidin-4-yl 2-ethylhexanoat, 1-Oxyl-2,2,6,6-tetramethylpiperidin-4-yl-Stearat, 1-Oxyl-2,2,6,6-tetramethylpiperidin-4-yl benzoat, 1-Oxyl-2,2,6,6-tetrame-thylpiperidin-4-yl 4-tert-butylbenzoat, Bis(1-oxyl-2,2,6,6-tetramethylpiperidin-4-yl)succinat, Bis(1-oxyl-2,2,6,6-tetramethylpiperidin-4-yl)adipat, Bis(1-oxyl-2,2,6,6-tetramethylpiperidin-4-yl)sebacat, Bis(1-oxyl-2,2,6,6-tetramethylpiperidin-4-yl)n-butylmalonat, Bis(1-oxyl-2,2,6,6-tetramethylpiperidin-4-yl)phthalat, Bis(1-oxyl-2,2,6,6-tetramethylpiperidin-4-yl)isophthalat, Bis(1-oxyl-2,2,6,6-tetramethylpiperidin-4-yl)terephthalat, Bis(1-oxyl-2,2,6,6-tetramethylpiperidin-4-yl)hexahydroterephthalat, N,N'-bis(1-oxyl-2,2,6,6-tetramethylpiperidin-4-yl)adipamid, N-(1-oxyl-2,2,6,6-tetramethylpiperidin-4-yl)-caprolactam, N-(1-oxyl-2,2,6,6-tetramethylpiperidin-4-yl)-dodecylsuccinimid, 2,4,6 Tris(1-oxyl-2,2,6,6-tetramethylpiperidin-4-yl) isocyanurat, 2,4,6Tris-[N-butyl-N-1-oxyl-2,2,6,6-tetramethylpiperidin-4-yl]-s-triazin, 4,4'-ethyl-enebis(1-oxyl-2,2,6,6-tetramethylpiperazin-3-on) und Kombinationen davon.

5. Zusammensetzung nach Anspruch 4, wobei das Nitroxylradikal-basierte Stabilisierungsmittel ausgewählt ist aus der Gruppe, bestehend aus 2,2,6,6-Tetramethyl-1-piperidinyloxy (TEMPO), 4-Hydroxy-2,2,6,6-tetramethyl-1-piperidinyloxy (4H-TEMPO) und Kombinationen daraus.

6. Verfahren zur Herstellung einer Zusammensetzung, definiert in einem der Ansprüche 1 bis 5, umfassend:

   Schritt a) Bereitstellen einer Benzen-enthaltenden Norbornadien-Lösung, umfassend mehr als 100 ppm Benzen,
   Schritt b) Leiten der Benzen-enthaltenden Norbornadien-Lösung durch ein Adsorptionsmittel wie z. B. ein Aktivkohlebett, Zeolith, Aluminiumoxid oder eine Kombination davon.
   Schritt c) Zugeben zur Norbornadien-Lösung, die aus Schritt b) stammt, eines Nitroxylradikal-basierten Stabilisierungsmittels.

7. Verfahren nach Anspruch 6, wobei das Adsorptionsmittel ein Aktivkohlebett ist.

8. Verfahren nach Anspruch 7, wobei das Aktivkohlebett Poren enthält, und wobei die Poren einen äquivalenten sphärischen Durchmesser aufweisen, der zwischen 0,4 und 50 nm liegt.

9. Verfahren nach Anspruch 7 oder 8, wobei die Aktivkohle bei einer Temperatur verwendet wird, die im Bereich von 0 °C bis 100 °C liegt, vorzugsweise 20 °C bis 50 °C, und einem Druck, der im Bereich von 80 kPa bis 665 kPa liegt, jedoch vorzugsweise zwischen 133 kPa und 200 kPa.

10. Verfahren nach einem der Ansprüche 7 bis 9, wobei die Aktivkohle anfänglich bei Temperaturen gebacken wird, die im Bereich von 30 °C bis 300 °C liegen, vorzugsweise unter 250 °C.

11. Verfahren nach einem der Ansprüche 7 bis 10, wobei die verwendete Aktivkohle durch Backen bei einer Temperatur regeneriert wird, die zwischen 50 °C und 400 °C vor der Wiederverwendung liegt.

12. Verfahren nach einem der Ansprüche 6 bis 11, wobei das behandelte Norbornadien, das aus Schritt b) stammt, dann durch Standarddestillierung gereinigt wird, um ein Endprodukt mit einer Reinheit zu erhalten, die größer als 99,9 %, vorzugsweise größer als 99,95 % ist.

**13.** Verwendung einer Zusammensetzung wie in einem der Ansprüche 1 bis 5 definiert in einem Dampfablageprozess zur Herstellung eines porösen Organosiliciumdioxid-Glasfilms, dargestellt durch die Formel $Si_vOwC_xH_yF_z$, wobei v+w+x+y+z = 100 %, v von 10 bis 35 Atomprozent ist, w von 10 bis 65 Atomprozent ist, x von 5 bis 30 Atomprozent ist, y von 10 bis 50 Atomprozent ist und z von 0 bis 15 Atomprozent ist, wobei das Verfahren Folgendes umfasst:

- Bereitstellen eines Substrats innerhalb einer Vakuumkammer;
- Einführen in die Vakuumkammer von gasförmigen Reagenzien, darin eingeschlossen mindestens einen Vorläufer, ausgewählt aus der Gruppe, bestehend aus einem Organosilan und einem Organosiloxan und einer Porogenzusammensetzung, wie in einem der Ansprüche 1 bis 5 definiert, die verschieden vom Vorläufer ist;
- Anwendung von Energie auf die gasförmigen Reagenzien in der Vakuumkammer, um eine Reaktion der gasförmigen Reagenzien hervorzurufen, um einen vorläufigen Film auf das Substrat abzulegen, wobei der vorläufige Film das Porogen enthält; und
- Entfernen vom vorläufigen Film im Wesentlichen des gesamten labilen organischen Materials, um den porösen Film mit Poren und einer dielektrischen Konstante von weniger als 2,6 bereitzustellen.

**14.** Verwendung nach Anspruch 13, wobei die Energie Plasmaenergie ist und das Porogen durch Aussetzen an ultraviolette Strahlung entfernt wird.

**15.** Verwendung nach Anspruch 14, wobei der mindestens eine Vorläufer ein Element ist, ausgewählt aus der Gruppe, bestehend aus Diethoxymethylsilan, Dimethoxymethylsilan, Di-isopropoxymethylsilan, Di-t-butoxymethylsilan, Methyltriethoxysilan, Methyltrimethoxysilan, Methyltriisopropoxysilan, Methyltri-t-butoxysilan, Dimethyldimethoxysilan, Dimethyldiethoxysilan, Dimethyldiisopropoxysilane, Dimethyldi-t-butoxysilan und Tetraethoxysilan.

## Revendications

**1.** Composition de porogène comprenant pour 100 % en poids :

- au moins 99,5 % en poids de norbornadiène ;
- de 10 ppm à 500 ppm en poids d'un agent de stabilisation à base d'un radical de nitroxyle ;
- de 250 ppb à 10 ppm en poids de benzène.

**2.** Composition selon la revendication 1 comprenant moins de 1 ppm de benzène.

**3.** Composition selon la revendication 1 ou la revendication 2 comprenant au moins 99,95 % de norbornadiène.

**4.** Composition selon l'une des revendications précédentes dans laquelle l'agent de stabilisation à base d'un radical de nitroxyle est sélectionné dans le groupe constitué du 2,2,6,6-tétraméthyl-1-pipéridinyloxy (TEMPO), du 4-hydroxy-2,2,6,6-tétraméthyl-1-pipéridinyloxy (4H-TEMPO), du di-tert-butyl nitroxyle, de la 1-oxyl-2,2,6,6-tétraméthylpipéridin-4-one, de l'acétate de 1-oxyl-2,2,6,6-tétraméthylpipéridin-4-yle, du 2-éthylhexanoate de 1-oxyl-2,2,6,6-tétraméthylpipéridin-4-yle, du stéarate de 1-oxyl-2,2,6,6-tétraméthylpipéridin-4-yle, du benzoate de 1-oxyl-2,2,6,6-tétraméthyl-pipéridin-4-yle, du 4-tert-butylbenzoate de 1-oxyl-2,2,6,6-tétraméthylpipéridin-4-yle, du succinate de bis(1-oxyl-2,2,6,6-tétraméthylpipéridin-4-yle), de l'adipate de bis(1-oxyl-2,2,6,6-tétraméthylpipéridin-4-yle), du sébacate de bis(1-oxyl-2,2,6,6-tétraméthyl-pipéridin-4-yle), du n-butylmalonate de bis(1-oxyl-2,2,6,6-tétraméthylpipéridin-4-yle), du phtalate de bis(1-oxyl-2,2,6,6-tétraméthylpipéridin-4-yle), de l'isophtalate de bis(1-oxyl-2,2,6,6-tétraméthylpipéridin-4-yle), du téréphtalate de bis(1-oxyl-2,2,6,6-tétra-méthylpipéridin-4-yle), de l'hexahydrotéréphtalate de bis(1-oxyl-2,2,6,6-tétraméthyl-pipéridin-4-yle), du N,N'-bis(1-oxyl-2,2,6,6-tétraméthylpipéridin-4-yle)adipamide, du N-(1-oxyl-2,2,6,6-tétraméthylpipéridin-4-yl)-caprolactame, du N-(1-oxyl-2,2,6,6-tétra-méthylpipéridin-4-yl)-dodécylsuccinimide, de l'isocyanurate de 2,4,6-tris(1-oxyl-2,2,6,6-tétraméthylpipéridin-4-yle), de la 2,4,6-tris-[N-butyl-N-1-oxyl-2,2,6,6-tétraméthyl-pipéridin-4-yl]-s-triazine, de la 4,4'-éthylènebis(1-oxyl-2,2,6,6-tétraméthylpipérazin-3-one) et de leurs combinaisons.

**5.** Composition selon la revendication 4, dans laquelle l'agent de stabilisation à base d'un radical de nitroxyle est sélectionné dans le groupe constitué du 2,2,6,6-tétra-méthyl-1-pipéridinyloxy (TEMPO), du 4-hydroxy-2,2,6,6-tétraméthyl-1-pipéridinyloxy (4H-TEMPO) et de leurs combinaisons.

**6.** Procédé de préparation d'une composition définie dans l'une quelconque des revendications 1 à 5 comprenant :

Étape a) fournir une solution de norbornadiène contenant du benzène comprenant plus de 100 ppm de benzène,
Étape b) passer la solution de norbornadiène contenant du benzène à travers un adsorbant tel qu'un lit de charbon actif, une zéolithe, l'alumine ou une combinaison de ceux-ci,
Étape c) ajouter à la solution de norbornadiène obtenue dans l'étape b) un agent de stabilisation à base d'un radical de nitroxyle.

**7.** Procédé selon la revendication 6, dans lequel l'adsorbant est un lit de charbon actif.

**8.** Procédé selon la revendication 7, dans lequel le lit de charbon actif contient des pores et dans lequel les pores présentent un diamètre sphérique équivalent compris entre 0,4 nm et 50 nm.

**9.** Procédé selon la revendication 7 ou 8, dans lequel le charbon actif est utilisé à une température située dans la plage allant de 0 °C à 100 °C, de préférence de 20 °C à 50 °C, et à une pression située dans la plage allant de 80 kPa à 665 kPa mais de préférence entre 133 kPa et 200 kPa.

**10.** Procédé selon l'une des revendications 7 à 9, dans lequel le charbon actif est initialement cuit à des températures situées dans la plage allant de 30 °C jusqu'à 300 °C, de préférence sous 250 °C.

**11.** Procédé selon l'une des revendications 7 à 10, dans lequel le charbon actif utilisé est régénéré par une cuisson à une température comprise entre 50 °C et 400 °C avant réutilisation.

**12.** Procédé selon l'une des revendications 6 à 11, dans lequel le norbornadiène traité obtenu dans l'étape b) est ensuite purifié par distillation classique pour obtenir un produit final ayant une pureté supérieure à 99,9 %, de préférence supérieure à 99,95 %.

**13.** Utilisation d'une composition telle que définie dans l'une des revendications 1 à 5 dans un procédé de dépôt en phase vapeur pour produire un film poreux en verre d'organosilice représenté par la formule $Si_vO_wC_xH_yF_z$, où v+w+x+y+z = 100 %, v va de 10 à 35 % atomique, w va de 10 à 65 % atomique, x va de 5 à 30 % atomique, y va de 10 à 50 % atomique et z va de 0 à 15 % atomique, ledit procédé comprenant :

- la fourniture d'un substrat au sein d'une chambre à vide ;
- l'introduction dans la chambre à vide de réactifs gazeux comprenant au moins un précurseur sélectionné dans le groupe constitué d'un organosilane et d'un organosiloxane, et une composition de porogène telle que définie dans l'une des revendications 1 à 5 qui est distincte du précurseur ;
- l'application d'une énergie aux réactifs gazeux dans la chambre à vide pour induire une réaction des réactifs gazeux afin de déposer un film préliminaire sur le substrat, dans lequel le film préliminaire contient le porogène ; et
- l'élimination à partir du film préliminaire de sensiblement la totalité de la substance organique labile pour fournir au film poreux des pores et une constante diélectrique inférieure à 2,6.

**14.** Utilisation selon la revendication 13, dans laquelle l'énergie est une énergie de plasma et le porogène est éliminé par exposition à un rayonnement ultraviolet.

**15.** Utilisation selon la revendication 14, dans laquelle l'au moins un précurseur est un membre sélectionné dans le groupe constitué du diéthoxyméthylsilane, du diméthoxyméthylsilane, du di-isopropoxyméthylsilane, du di-t-butoxyméthylsilane, du méthyltriéthoxysilane, du méthyltriméthoxysilane, du méthyltriisopropoxysilane, du méthyltri-t-butoxysilane, du diméthyldiméthoxysilane, du diméthyldiéthoxysilane, du diméthyldi-isopropoxysilane, du diméthyldi-t-butoxysilane, et du tétraéthoxysilane.

**Figure 1: Control : Raw BCHD material**

The benzene peak can clearly be seen at 8,177minutes.

Benzene was totally removed
[Benzene]<250ppb (DL)

**Figure 2:**
**GC of BCHD after passing through activated coal**

**Figure 3 : GC of Benzene-containing BCHD, after purification**
**GC of Benzene-free BCHD, after purification**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6756085 B
- US 20090159843 A
- US 5458741 A

**Non-patent literature cited in the description**

- *ESPR - Environ Sci & Pollut Res,* 2003, vol. 10 (1), 6-8